# FASCICULE DE BREVET EUROPEEN

(11) **EP 0 646 002 B1**
(45) Date de publication et mention de la délivrance du brevet: **27.12.1995**
(21) Numéro de dépôt: 93913148.8
(22) Date de dépôt: 16.06.1993
(51) Int. Cl.: A61K 9/51

(54) **PREPARATION ET APPLICATION DE NOUVEAUX SYSTEMES COLLOIDAUX NANOVESICULAIRES DISPERSIBLES A BASE DE CYCLODEXTRINE, SOUS FORME DE NANOCAPSULES**
HERSTELLUNG UND ANWENDUNG VON AUF BASIS VON CYCLODEXTRIN DISPERGIERBAREN KOLLOIDALEN VESIKULÄR-SYSTEMEN, IN FORM VON NANOKAPSELN
PREPARATION AND USE OF NOVEL CYCLODEXTRIN-BASED DISPERSIBLE NANOVESICULAR COLLOIDAL SYSTEMS IN THE FORM OF NANOCAPSULES

(30) Priorité: 16.06.1992 FR 9207285
(43) Date de publication de la demande: 05.04.1995
(73) Titulaire: CENTRE NATIONAL DE LA RECHERCHE SCIENTIFIQUE (CNRS), 75007 Paris (FR)
(72) Inventeur: SKIBA, Mohamed, F-91370 Verrières-le-Buisson (FR); WOUESSIDJEWE, Denis, F-92160 Antony (FR); FESSI, Hatem, F-75014 Paris (FR); DEVISSAGUET, Jean-Philippe, F-92200 Neuilly-sur-Seine (FR); DUCHENE, Dominique, F-75116 Paris (FR); PUISIEUX, Francis, F-94700 Maisons-Alfort (FR)
(74) Mandataire: Le Guen, Gérard
(86) Numéro de dépôt international: FR9300593
(87) Numéro de publication internationale: WO9325194

(56) Documents cités:
- EP-A- 0 274 961
- EP-A- 0 451 082
- FR-A- 2 551 072

## Description

La présente invention a pour objet la préparation et l'application d'un nouveau système colloïdal nanovésiculaire dispersible à base de cyclodextrine sous forme de particules sphériques de type vésiculaire de taille allant de 100 à 900 nm (nanocapsules), contenant une molécule active.

Des systèmes nanovésiculaires, comprenant des nanocapsules et constitués d'un noyau central solide ou liquide enveloppé par une membrane continue insoluble ont déjà été décrits dans la littérature. Les constituants de la membrane peuvent être des macromolécules synthétiques (polymères), naturelles (protéines) ou des lipides (liposomes).

Des particules de polymère, submicroniques sont déjà connues notamment d'après les brevets BE-A-808 034, BE-A-839 748, BE-A-869 107, FR-A-2 504 408, FR-A-2 515 960 et EP-A-0 274 961.

Selon BE-A-808 034 et -839 748, les particules submicroniques sont formées par polymérisation micellaire d'un monomère tel qu'un dérivé de l'acide acrylique. De même le BE-A-869 107, FR-A-2 504 408 et FR-A-2 515 960 décrivent la préparation de nanocapsules biodégradables obtenues par polymérisation d'un cyanoacrylate d'alkyle et contenant une substance biologiquement active. Les méthodes font appel à une polymérisation en solution et sont donc limitées à l'utilisation d'un nombre limité de polymères pouvant être obtenus notamment par vinyladdition et ne convient pas à des polymères naturels ou hémisynthétiques. De plus il est difficile de maîtriser le poids moléculaire constitutif des nanocapsules et il est nécessaire, notamment en vue d'usage biologique, d'éliminer les monomères et oligomères résiduels, le cas échéant les réactifs de polymérisation (initiateur et catalyseur) en excès, ainsi que les surfactifs s'ils sont utilisés à forte concentration ou ne sont pas biocompatibles. Or la purification des nanocapsules, vu leur taille, n'est pas toujours possible.

On a aussi décrit la préparation de nanocapsules protéiques, notamment de gélatine. Ainsi, Krause et coll. (Pharm. Research, 239 (1985)), obtiennent des nanocapsules de gélatine dont le contenu est lipophile (chloroforme), à partir d'une émulsion de chloroforme dans une phase continue aqueuse (solution d'albumine). Les nanocapsules sont ensuite durcies par le glutaradéhyde, dont l'excès doit être détruit par du métabisulfite de sodium qui doit être éliminé. De plus, la stabilité du chloroforme dans la nanocapsule semble limitée par son évaporation : les nanocapsules peuvent se vider du solvant, mais conservent le produit lipophile initialement en solution dans le chloroforme.

EP-A-0 274 961 décrit un procédé de préparation de nanocapsules ayant une paroi polymère contenant une substance, par une méthode de désolvatation consistant à mélanger deux phases de solvants, l'un étant non-solvant de la substance mais soluble dans l'autre solvant.

Le document FR-A-2 551 072 décrit la préparation de microcapsules à libération prolongée.

Selon la présente invention, on propose comme matière de base des cyclodextrines modifiées préparées par acylation de cyclodextrines naturelles. Elles ont l'avantage d'être biodégradables, leur administration est suivie d'une libération de la molécule active, et il est possible d'obtenir une biodégradabilité, convenablement programmée en faisant appel à des cyclodextrines modifiées différant entre elles par la nature de la chaîne alkyle du groupe acyle utilisé.

De telles cyclodextrines modifiées et leur préparation sont notamment décrites par Ping Zhang, Chang-Chun Ling, A.W Coleman, H.Parrot-Lopez et H.Galons dans Tetr. Lett. 32, n°24 pp.2769-70, 1991.

L'invention a pour objet un procédé de préparation d'un système colloïdal dispersible à base de cyclodextrine sous forme de nanocapsules, caractérisé en ce que
1) on prépare une phase liquide constituée essentiellement par une solution de cyclodextrine modifiée par des groupes acyle et d'une huile dans un solvant ou mélange de solvants organique(s) contenant ou non un surfactif et pouvant être additionnée d'une molécule active,
2) on prépare une seconde phase liquide constituée essentiellement par de l'eau ou un mélange aqueux, contenant ou non un surfactif et pouvant être additionnée d'une molécule active, et
3) on ajoute sous agitation modérée, l'une des phases liquides obtenues sous (1) ou (2) à l'autre, de manière à obtenir pratiquement instantanément une suspension colloïdale de nanocapsules dont la paroi est constituée de cyclodextrine modifiée et la cavité est constituée d'huile contenant le cas échéant la molécule active.

Si l'on désire, on élimine tout ou une partie du solvant ou du mélange de solvants et de l'eau ou du mélange aqueux, de manière à obtenir une suspension colloïdale de concentration voulue en nanocapsules ou une poudre de nanocapsules.

La cyclodextrine modifiée utilisée selon l'invention est notamment une cyclodextrine dont les groupes hydroxy, de préférence les hydroxy secondaires de chaque unité glucose la formant ont été estérifiés par un groupe acyle aliphatique ou aromatique pouvant être substitués par un ou plusieurs groupements fonctionnels, telle qu'une béta-cyclodextrine acylée par un groupe alcanoyle de 2 à 20 atomes de carbone, notamment 6 à 14 atomes de carbone. Ces produits sont décrits par Ping Zhang et coll. précité.

La molécule active peut être un principe médicamenteux, un réactif biologique, un principe cosmétique, ou un produit chimique. L'invention permet d'obtenir des nanocapsules de cyclodextrine modifiée contenant l'huile (utilisables telles quelles) ou comprenant de plus cette molécule active.

Le solvant organique dans la phase (1) peut être un alcool tel que méthanol, éthanol, isopropanol, etc... ou une cétone telle que l'acétone.

L'huile peut être une huile végétale ou minérale, ou toute substance huileuse, par exemple l'huile d'olive, le benzoate de benzyle, le myristate d'isopropyle, des glycérides d'acide gras, (p.ex. un Miglyol (R)), l'huile de bouquet ou autre. La proportion d'huile/cyclodextrine modifiée est de préférence entre 1/1 et 100/1 V/P.

L'eau ou un mélange aqueux (eau salée, acidifiée, basifiée etc...) est le non-solvant dans la phase (2).

Le procédé peut être réalisé à différentes températures (qui influent peu sur son déroulement), notamment entre 0°C et la température d'ébullition des solvants.

Le rapport des volumes phase (1)/phase (2) peut varier préférentiellement de 0,1 à 1.

Le(s) surfactif(s) est (sont) présent(s) notamment en une proportion de 0,1 à 10 %, de préférence 0,2 à 2 % en poids de la suspension colloïdale obtenue dans l'étape 3.

On entend par agitation modérée une agitation suffisante pour homogénéiser le mélange des phases (1) et (2), par exemple au moyen d'un barreau magnétique à 50-500 rpm, par exemple 100 rpm. Elle n'est pas indispensable pour de faibles quantités de produit.

Finalement, la suspension colloïdale de nanocapsules peut être à volonté concentrée, stérilisée, tamponnée (par exemple au pH physiologique), et lyophilisée.

L'invention permet d'obtenir des nanocapsules de cyclodextrines de 100 à 900 nm notamment de 100 à 500 nm selon les conditions opératoires.

Les nanocapsules de la présente invention contrairement aux nanocapsules formées seulement de polymère, présentent l'avantage de permettre un taux d'incorporation nettement plus élevé. Ceci est dû à la possibilité de double charge, en premier lieu, une charge au sein de la capsule et en second lieu dans la cavité de la cyclodextrine à condition que la molécule invitée ait une conformation convenable par rapport à la cavité.

De plus, pour les nanocapsules faisant l'objet de la présente invention, la préparation a l'avantage d'être réversible, on peut solubiliser les nanocapsules et préparer les nanocapsules à partir de cette solution suivant le mode opératoire.

Les nanocapsules obtenues suivant l'invention se présentent au microscope électronique à transmission, après cryofracture sous la forme de capsules sphériques.

L'enveloppe externe des nanocapsules contenant une huile ou une substance active dispersée dans une huile apparaît sous forme de paroi d'épaisseur très régulière de l'ordre de quelques nanomètres. La paroi n'est pas formée par polymérisation.

Le taux d'encapsulation est toujours très élevé dans le cas de produits lipophiles ; 200 mg de cyclodextrine modifiée permet par exemple d'encapsuler 3 ml de benzoate de benzyle ou 230 mg de progestérone et 0,6 ml d'huile utilisée comme support.

Les substances actives insolubles dans les solvants organiques, et solubles dans l'eau peuvent également être encapsulées. Il suffit qu'elles présentent une certaine affinité pour la phase lipophile ; le procédé de préparation des nanocapsules est analogue. Il suffit de solubiliser la substance active dans la phase aqueuse. Le taux d'encapsulation dépend du coefficient du partage de la substance active et de sa lipophilie.

Les nanocapsules de l'invention, contenant une huile, sont stables à l'autoclavage pendant plus de 15 min. à 120°C.

Les nanocapsules de l'invention sont stables à l'ultracentrifugation. Après une ultracentrifugation à 220 000 g pendant 2 h 30 min., la majeure partie des nanocapsules peut être facilement redispersée dans l'eau.

Les résultats précédents ne peuvent s'expliquer que par la présence d'une paroi enveloppant le matériel encapsulé et que par encapsulation complète du principe actif.

La substance active, contenue dans les nanocapsules de l'invention, peut être par exemple une molécule médicamenteuse à usage humain ou vétérinaire ou un produit pour le diagnostic. Comme molécule médicamenteuse, on peut citer plus particulièrement les produits chimiques doués de propriétés pharmacologiques et par exemple, les substances antimitotiques ou antinéoplasiques comme la méthotréxate, l'actinomycine D, l'adriamycine, la daunorubicine, la bléomycine, et la vincristine ou les substances antibiotiques comme les pénicillines, les céphalosphorines et l'acide nalidixique, les antibiotiques du type aminoglycoside et ceux de la famille de la virginiamycine et les substances hormonales, notamment les hormones stéroïdiennes. Ces molécules médicamenteuses peuvent être notamment des composés chimiques à haut poids moléculaire comme l'insuline et l'héparine et l'expression "molécule médicamenteuse" comprend également des produits biologiques comme les antigènes, les enzymes, les protéines, les virus ou des constituants de virus, de bactéries ou de cellules. Les nanocapsules suivant l'invention peuvent également contenir un produit pour le diagnostic comme par exemple la fluorescéine et la séralbumine humaine radio-active. Les nanocapsules suivant l'invention peuvent également contenir un produit pour le dignostic, notamment des produits radio-opaques lipophiles tels que les huiles iodées.

En médecine humaine ou vétérinaire, les nanocapsules de l'invention peuvent être utilisées comme vecteurs de médicaments administrés avec ou sans excipient adéquat par voie orale, sous cutanée, intradermique, intramusculaire ou intraveineuse et leur diffusion dans les tissus les rend particulièrement intéressantes pour les traitements par voie générale.

**Théorie de l'encapsulation** : Dans le solvant par exemple dans l'ethanol absolu, la substance huileuse et la cyclodextrine forment une solution vraie. La cyclodextrine modifiée possède des propriétés tensioactives grâce à sa structure particulière. L'huile est complètement lipophile. Le stade important de la préparation est l'injection lente de la solution contenant l'huile et la cyclodextrine dans la phase aqueuse. L'alcool absolu possède une affinité considérable pour l'eau et, au moment du contact, il se mélange rapidement avec cette dernière en dégageant une certaine quantité de chaleur (appelée chaleur du mélange). La substance huileuse précipite en formant des petites particules de l'ordre du nanomètre. A ce moment les molécules de cyclodextrine s'orientent grâce à leur polarité en formant un film qui enrobe complètement les nanoparticules d'huile. En présence d'eau le film est constitué par les têtes hydrophiles des molécules de cyclodextrine stabilisées par des liaisons hydrogène à la surface des nanoparticules. Le rôle du surfactif utilisé est plus ou moins important. Il est possible d'obtenir des nanocapsules dans de l'eau sans surfactif. Toutefois les nanocapsules ainsi obtenues s'agglomèrent rapidement dans le cas ou il est intéressant de concentrer, en formant des agrégats difficiles à redisperser.

L'invention est illustrée par les exemples suivants :

### Exemple 1

Préparation de nanocapsules de cyclodextrine modifiée à 6 carbone.

On utilise une béta-cyclodextrine dont les OH secondaires des unités glucose le formant ont été estérifiés par des groupes hexanoyle, préparée selon Ping Zhang et coll.

| Phase 1 | |
|---|---|
| béta-cyclodextrine modifiée à 6 C | 50 mg |
| acétone | 50 ml |
| benzoate de benzyle | 0,5 ml |

| Phase 2 | |
|---|---|
| Pluronic (R) F68 | 62,5 mg |
| eau déminéralisée ou distillée | 25 ml |

La phase 1 est ajoutée sous agitation magnétique à la phase 2. Le milieu devient immédiatement opalescent par formation de nanocapsules de cyclodextrine modifiée. La taille moyenne des nanocapsules mesurée par un diffractomètre à rayon laser (Nanosizer (R) de chez Coultronics) est de 300 nm avec un indice moyen de dispersion de 0,08.

La suspension peut être concentrée sous pression réduite au volume désiré, par exemple 5 ml ou plus ou moins.

Après un repos prolongé (14 mois), l'aspect de la suspension de nanocapsules demeure inchangé et on n'observe, en particulier, ni sédimentation irréversible, ni variation de la taille des nanocapsules.

### Exemple 2 : (variante de l'exemple 1)

On procède comme dans l'exemple 1, mais en ajoutant la phase aqueuse à la phase acétonique. Les nanocapsules obtenues présentent les mêmes caractéristiques que dans l'exemple 1.

### Exemple 3 : (variante de l'exemple 1)

On procède comme dans l'exemple 1, mais en ajoutant la phase acétonique à la phase aqueuse sans agitation du milieu. Les nanocapsules obtenues ont une taille de 300 nm avec un indice moyen de dispersion 0,5.

### Exemple 4 : (variante de l'exemple 1)

On procède comme dans l'exemple 1, mais sans ajouter d'agent de surface à la phase aqueuse. Les nanocapsules ont une taille de 500 nm avec un indice moyen de dispersion de 0,6.

### Exemple 5

Préparation stérile de nanocapsules de cyclodextrine modifiée à 6 carbone.

On procède comme dans l'exemple 1, puis la suspension est stérilisée à l'autoclave à 120°C pendant 15 minutes. La taille moyenne des particules demeure pratiquement inchangée après stérilisation.

### Exemple 6

Préparation lyophilisée de nanocapsules de cyclodextrine modifiée à 6 carbone.

On procède comme dans l'exemple 1, puis la suspension est lyophilisée. L'addition d'un cryoprotecteur (maltose, tréhalose...etc) n'est pas indispensable. La taille moyenne des particules mesurée juste après la lyophilisation demeure inchangée.

### Exemple 7

Préparation de nanocapsules de cyclodextrine modifiée à 12 carbone.

On procède comme dans l'exemple 1, en remplaçant la cyclodextrine modifiée à 6 carbone par la cyclodextrine modifiée à 12 carbone, c'est-à-dire acylée par des groupes dodécanoyle préparée selon P. Zhang et coll. La taille moyenne des nanocapsules est de 300 nm avec un indice moyen de dispersion de 0,1. Les nanocapsules peuvent être stérilisées à l'autoclave et lyophilisées comme celles avec 6 carbone.

### Exemple 8 :

Préparation de nanocapsules de cyclodextrine modifiée à 14 carbones.
On procède comme dans l'exemple 1, en remplaçant la cyclodextrine modifiée à 6 carbone par la cyclodextrine modifiée à 14 carbone, c'est à dire acylée par des groupes tetradécanoyle. La taille moyenne des nanocapsules est de 110 nm avec un indice moyen de dispersion de 0,1.
Les nanocapsules de cyclodextrine modifiée à 14 carbone peuvent être stérilisées à l'autoclave ou autre et lyophilisées comme celles avec 6 carbone.

### Exemple 9

Stabilité des nanocapsules de cyclodextrine en présence de forces ioniques variables.
On procède comme indiqué dans l'exemple 1.
Après concentration de la suspension de nanocapsules de cyclodextrine jusqu'à un volume de 10 ml, on ajoute progressivement à celle-ci des quantités croissantes de chlorure de sodium. La suspension de nanocapsules est parfaitement stable lorsque la concentration en chlorure de sodium correspond à l'isotonie avec le sang et le demeure jusqu'à une concentration supérieure à 3 fois la concentration isotonique.

### Exemple 10

Stabilité des nanocapsules de cyclodextrine en présence d'un milieu acide ou basique.
On procède comme indiqué dans l'exemple 1.
Après concentration de la suspension de nanocapsules de cyclodextrine jusqu'à un volume de 10 ml, on ajoute progressivement à celle-ci des quantités croissantes d'acide chlorydrique (1N) ou de la soude (1N). La suspension de nanocapsules est parfaitement stable.

### Exemple 11

Stabilité des nanocapsules de cycodextrine à la température.
On procède comme indiqué dans l'exemple 1.
Après concentration de la suspension de nanocapsules de cyclodextrine jusqu'à un volume de 10 ml, on place chaque lot à 4°C, 25°C et 40°C.
Les suspensions demeurent stables dans le temps et ne présentent, après 14 mois de conservation, ni sédimentation irréversible, ni variation de la taille des nanocapsules.

### Exemple 12

Préparation de nanocapsules en présence d'un sel.
On procède comme indiqué dans l'exemple 1, mais la phase aqueuse est additionnée de 90 mg de chlorure de sodium. Après concentration de la suspension de nanocapsules jusqu'à un volume de 10 ml, correspondant compte tenu du chlorure de sodium à l'isotonie avec le sang, les nanocapsules ont une taille moyenne de 320 nm avec un indice moyen de dispersion de 1.
La suspension demeure stable dans le temps et ne présente, après 14 mois de conservation, ni sédimentation irréversible, ni variation de la taille des nanocapsules

### Exemple 13

Addition de non-solvant dans la phase du solvant.
On procède comme dans l'exemple 1, la cyclodextrine est dissoute dans un mélange acétone/eau (90/10, v/v), au lieu d'acétone pure. La présence d'une faible proportion de non-solvant de la cyclodextrine dans un solvant, conduit à des nanocapsules dont la taille moyenne est de 380 nm avec un indice moyen de dispersion de 0,5.

### Exemple 14

Stabilité des nanocapsules de cyclodextrine aux ultrasons.
On procède comme dans l'exemple 1. Après concentration de la suspension de nanocapsules de cyclodextrine jusqu'à un volume de 10 ml, on place la suspension de nanocapsules de cyclodextrine dans un bain à ultrasons pendant trois heures.
La suspension demeure stable dans le temps et ne présente, après 14 mois de conservation, ni sédimentation irréversible, ni variation de la taille des nanocapsules.

### Exemple 15

Préparation de nanocapsules en présence d'un principe actif hydrophile.
On procède selon l'exemple 1, mais on ajoute 10 mg de doxorubicine dans la phase aqueuse. Dans la phase 2 on utilise de l'éthanol et 0,5 ml de benzoate de benzyle. Les nanocapsules obtenues ont une taille moyenne de 300 nm et un indice moyen de dispersion de 1. Après ultracentrifugation et dosage de la doxorurubicine dans la phase dispersante, la quantité de principe actif incorporé dans les nanocapsules représente 60% de la quantité initiale.

### Exemple 16

Préparation de nanocapsules en présence d'un principe actif lipophile.
On procède comme dans l'exemple 1, mais on ajoute 30 mg de d'indométacine et 0,3 ml de benzoate de benzyle dans la phase acétonique. Les nanocapsules obtenues ont une taille moyenne de 320 nm et un indice de dispersion de 0,5. Après ultracentrifugation et dosage de l'indométacine dans la phase dispersante, la quantité de principe actif incorporé dans les nanocapsules représente 90% de la quantité initiale.

### Exemple 17

Préparation de nanocapsules contenant de la progestérone.
On procède comme dans l'exemple 1, mais on ajoute 230 mg de progestérone et 0,6 ml de benzoate de benzyle dans la phase 1. Les nanocapsules obtenues ont une taille moyenne de 120 nm et un indice de dispersion de 0,2. Après ultracentrifugation et dosage de la progestérone dans la phase dispersante, la quantité de principe actif incorporé dans les nanocapsules représente 90% de la quantité initiale.

### Exemple 18

Préparation de nanocapsules contenant un colorant lipophile le soudan III.
On procède comme dans l'exemple 1, mais on ajoute 5mg de soudan III dans la phase I. Une faible quantité est précipitée et reste sur le filtre. Les nanocapsules obtenues ont une taille moyenne de 130 nm et un indice de dispersion de 0,2.

Les nanocapsules obtenues selon l'invention peuvent trouver des applications dans de nombreux secteurs techniques.

En tant que "**vecteurs**" de principe actif, en thérapeutique humaine et animale les nanocapsules permettent d'envisager :
d'atteindre de nouveaux sites d'action, en particulier intracellulaires, voire intralysosomiaux ;
d'utiliser de nouvelles voies d'administration pour les principes actifs connus, en augmentant la stabilité et/ou l'absorption des principes actifs, ou en permettant la réalisation de formes injectables par voie intravasculaire, de principes actifs insolubles ;
de modifier la distribution tissulaire des principes actifs, par un meilleur ciblage vers des sites d'actions favorables et/ou un détournement des sites d'effets indésirables voire toxiques (amélioration de l'index thérapeutique).

En **pharmacie**, ces dispersions colloïdales de cyclodextrine peuvent permettre notamment :
de réaliser des formes injectables de médicaments insolubles,
de stabiliser un principe actif médicamenteux...etc.

Dans le domaine de la **phytopharmacie** les nanocapsules peuvent véhiculer des insecticides, des pesticides, etc. Leur taille peut permettre d'envisager une action plus puissante par une meilleure pénétration à travers la cuticule. La faible viscosité de la dispersion autorise une pulvérisation très facile sous forme de goutellettes de très petite taille plus efficaces car plus couvrantes.

En **cosmétologie**, les nanocapsules de cyclodextrine peuvent transporter des produits anti-radicalaires ou autres au niveau du derme.

Dans le domaine des **peintures**, **vernis et traitements des surfaces** d'une manière générale, les nanocapsules permettent de véhiculer des pigments, des réactifs, des décapants sous forme de dispersion aqueuse de très faible viscosité, aisée à pulvériser ou à appliquer, et qui peut, si nécessaire, être rendue visqueuse, voire adhésive (remise en suspension des nanosphères dans un véhicule approprié). La taille réduite des nanosphères conduit à une très grande finesse du dépôt et à une très grande homogénéité, par exemple de pigmentation.

Les nanocapsules obtenues selon l'invention peuvent être utilisées dans les domaines de **l'imprimerie** et de la **reprographie**, du **traitement de surface des textiles** et **des fibres**, ou autre, de la **photographie**, de la **lubrification**, de **l'agriculture**.

## Revendications

1. Procédé de préparation d'un système colloïdal dispersible à base de cyclodextrine sous forme de nanocapsules, caractérisé en ce que
1) on prépare une phase liquide constituée par une solution de cyclodextrine modifiée par des groupes acyle et d'une huile dans un solvant ou mélange de solvants organique(s) choisi(s) parmi le méthanol, l'éthanol, l'isopropanol et l'acétone contenant ou non un surfactif et pouvant être additionnée d'une molécule active,
2) on prépare une seconde phase liquide constituée par de l'eau ou un mélange aqueux, contenant ou non un surfactif et pouvant être additionnée d'une molécule active, et
3) on ajoute sous agitation modérée, c-à-d sous agitation suffisante pour homogénéiser le melange, l'une des phases liquides obtenues sous (1) ou (2) à l'autre, de manière à obtenir instantanément une suspension colloïdale de nanocapsules dont la paroi est constituée de cyclodextrine modifiée et la cavité est constituée d'huile contenant le cas échéant la molécule active.

2. Procédé selon la revendication 1, caractérisé en ce que l'on élimine tout ou partie du solvant ou du mélange de solvants et de l'eau ou du mélange aqueux de manière à obtenir une suspension colloïdale de concentration voulue en nanocapsules ou à obtenir un poudre de nanocapsules.

3. Procédé selon la revendication 1 ou 2, caractérisé en ce que ledit solvant de la phase (1) est choisi parmi les alcools et les cétones ou leurs mélanges.

4. Procédé selon la revendication 1, caractérisé en ce que la cyclodextrine modifiée est une béta-cyclodextrine estérifiée par des groupes alcanoyle de 2 à 20 atomes de carbone.

5. Procédé selon la revendication 1, caractérisé en ce que la cyclodextrine modifiée est une béta-cyclodextrine estérifiée par des groupes alcanoyle de 6 à 14 atomes de carbone.

6. Procédé selon l'une des revendications 1 à 5, caractérisé en ce que la molécule active est un principe actif médicamenteux ou un précurseur médicamenteux à usage humain ou vétérinaire, un réactif biologique ou un principe cosmétique, un virus, un constituant de virus, de bactérie ou de cellule, un antigène, un allergène ou une enzyme.

7. Procédé selon l'une quelconque des revendications 1 à 6, caractérisé en ce que le rapport des volumes phase (1)/phase (2) est de 0,1 à 1.

8. Procédé selon la revendication 2, caractérisé en ce que la totalité de l'eau est éliminée par lyophilisation.

9. Procédé selon l'une quelconque des revendications 1 à 8, caractérisé en ce que le(s) surfactif(s) est (sont) présent(s) en une proportion de 0,1 à 10%, de préférence 0,2 à 2% en poids de la suspension colloïdale obtenue dans l'étape 3.

10. Utilisation des nanocapsules de cyclodextrine modifiée obtenues suivant l'une quelconque des revendications 1 à 9 comme vecteurs de principes actifs médicamenteux ou cosmétiques.

11. Utilisation des nanocapsules de cyclodextrine modifiée obtenues suivant l'une quelconque des revendications 1 à 9 comme vecteur de produits chimiques.

## Claims

1. A process for the preparation of a cyclodextrin-based dispersible colloidal system in the form of nanocapsules, characterised in that
1) a liquid phase is prepared, comprising a solution of cyclodextrin modified by acyl groups and an oil in an organic solvent or mixture of organic solvents selected from methanol, ethanol, isopropanol and acetone, optionally containing a surfactant and to which an active molecule can be added,
2) a second liquid phase is prepared, comprising water or an aqueous mixture, optionally containing a surfactant and to which an active molecule can be added, and
3) one of the liquid phases obtained under (1) or (2) is added to the other while stirring gently, that is to say stirring sufficiently to homogenise the mixture, so as to obtain instantaneously a colloidal suspension of nanocapsules, the wall thereof being formed by modified cyclodextrin and the cavity consisting of oil optionally containing the active molecule.

2. A process according to claim 1, characterised in that all or part of the solvent or mixture of solvents and the water or aqueous mixture is removed so as to obtain a collodial suspension of the required concentration of nanocapsules or to obtain a nanocapsule powder.

3. A process according to claim 1 or 2, characterised in that the said solvent of phase (1) is selected from the alcohols and the ketones or mixtures thereof.

4. A process according to claim 1, characterised in that the modified cyclodextrin is a betacyclodextrin esterified by alkanoyl groups of 2 to 20 carbon atoms.

5. A process according to claim 1, characterised in that the modified cyclodextrin is a betacyclodextrin esterified by alkanoyl groups of 6 to 14 carbon atoms.

6. A process according to any one of claims 1 to 5, characterised in that the active molecule is a medicinal active principle or a medicinal precursor for human or veterinary use, a biological reagent or a cosmetic principle, a virus, a viral, bacterial or cellular constituent, an antigen, an allergen or an enzyme.

7. A process according to any one of claims 1 to 6, characterised in that the phase (1)/phase (2) volume ratio is from 0.1 to 1.

8. A process according to claim 2, characterised in that all the water is removed by lyophilisation.

9. A process according to any one of claims 1 to 8, characterised in that the surfactant(s) is (are) present in a proportion of 0.1% to 10%, preferably 0.2% to 2% by weight of the colloidal suspension obtained in stage (3).

10. Use of the modified cyclodextrin nanocapsules obtained according to any one of claims 1 to 9 as carriers of medicinal or cosmetic active principles.

11. Use of the modified cyclodextrin nanocapsules obtained according to any one of claims 1 to 9 as a carrier of chemical products.

## Patentansprüche

1. Verfahren zur Herstellung eines dispergierbaren kolloidalen Systems auf der Grundlage von Cyclodextrin in Form von Nanokapseln, **dadurch gekennzeichnet**, daß man
1) eine flüssige Phase aus einer Lösung von mit Acylgruppen modifiziertem Cyclodextrin und einem Öl in einem organischen Lösungsmittel oder einer Mischung von organischen Lösungsmitteln, ausgewählt aus Methanol, Ethanol, Isopropanol und Aceton, welches bzw. welche gegebenenfalls ein oberflächenaktives Mittel enthält und mit einem aktiven Molekül versetzt werden kann, herstellt,
2) eine zweite flüssige Phase aus Wasser oder einer wäßrigen Mischung, die gegebenenfalls ein oberflächenaktives Mittel enthält und mit einem aktiven Molekül versetzt werden kann, herstellt und
3) unter mäßiger Bewegung, d. h. ausreichender Bewegung zur Homogenisierung der Mischung eine der beiden unter (1) oder (2) erhaltenen flüssigen Phasen zu der anderen Phase gibt in der Weise, daß man augenblicklich eine kolloidale Suspension von Nanokapseln erhält, deren Wandung aus modifiziertem Cyclodextrin gebildet ist und deren Innenraum aus einem Öl gebildet ist, das gegebenenfalls ein aktives Molekül enthält.

2. Verfahren nach Anspruch 1, **dadurch gekennzeichnet**, daß man das gesamte oder einen Teil des Lösungsmittels oder der Lösungsmittelmischung und des Wassers oder der wäßrigen Mischung entfernt, so daß man eine kolloidale Suspension mit der gewünschten Konzentration von Nanokapseln oder ein Nanokapselpulver erhält.

3. Verfahren nach Anspruch 1 oder 2, **dadurch gekennzeichnet**, daß das Lösungsmittel der Phase (1) aus Alkoholen und Ketonen oder Mischungen davon ausgewählt ist.

4. Verfahren nach Anspruch 1, **dadurch gekennzeichnet**, daß das modifizierte Cyclodextrin ein mit Alkanoylgruppen mit 2 bis 20 Kohlenstoffatomen verestertes Beta-Cyclodextrin ist.

5. Verfahren nach Anspruch 1, **dadurch gekennzeichnet**, daß das modifizierte Cyclodextrin ein mit Alkanoylgruppen mit 6 bis 14 Kohlenstoffatomen verestertes Beta-Cyclodextrin ist.

6. Verfahren nach einem der Ansprüche 1 bis 5, **dadurch gekennzeichnet**, daß das aktive Molekül ein Arzneimittelwirkstoff oder ein Arzneimittelvorläufer zur Verwendung beim Menschen oder beim Tier ist, ein biologisches Reagens oder ein kosmetischer Wirkstoff, ein Virus, ein Virusbestandteil, ein Bakterium oder eine Zelle, ein Antigen, ein Allergen oder Enzym ist.

7. Verfahren nach einem der Ansprüche 1 bis 6, **dadurch gekennzeichnet**, daß das Phase (1)/Phase /(2)-Volumenverhältnis 0,1 bis 1 beträgt.

8. Verfahren nach Anspruch 2, **dadurch gekennzeichnet**, daß die Gesamtheit des Wassers durch Gefriertrocknen entfernt wird.

9. Verfahren nach einem der Ansprüche 1 bis 8, **dadurch gekennzeichnet**, daß das (die) oberflächenaktive(n) Mittel in einer Menge von 0,1 bis 10 Gew.-%, vorzugsweise 0,2 bis 2 Gew.-% in der in der Stufe 3 erhaltenen kolloidalen Suspension vorhanden ist (sind).

10. Verwendung der nach einem der Ansprüche 1 bis 9 erhaltenen Nanokapseln aus modifiziertem Cyclodextrin als Vektoren für medikamentöse oder kosmetische Wirkstoffe.

11. Verwendung der nach einem der Ansprüche 1 bis 9 erhaltenen Nanokapseln aus modifiziertem Cyclodextrin als Vektor für chemische Produkte.
